# EUROPEAN PATENT APPLICATION

(11) **EP 4 438 736 A1**
(43) Date of publication of application: **02.10.2024**
(21) Application number: 23164790.0
(22) Date of filing: 28.03.2023
(51) Int. Cl.: C12P 17/12, C12N 9/10, C12N 9/88, C12N 15/52, A61K 31/00, A23K 10/12, C12P 13/04

(54) **METHOD FOR PRODUCING ECTOINE**

(71) Applicant: 350 PPM Biotech GmbH, 20148 Hamburg (DE)
(72) Inventor: MACK, Matthias, 68723 Oftersheim (DE); KASPAR, Charlotte, 69221 Dossenheim (DE); KACHEL, Benjamin, 68165 Mannheim (DE)
(74) Representative: Uexküll & Stolberg

(57) **Abstract**

The present invention generally relates to the field of producing ectoine in a bacterial organism. In particular, the invention relates to a method that consumes carbon dioxide and comprises culturing a chemolithoautotrophic bacterium which belongs to the genus *Hydrogenovibrio* under suitable conditions to produce ectoine. The ectoine produced by the chemolithoautotrophic bacterium according to the method of the present invention is secreted into the culture medium and can be readily obtained therefrom. In another aspect, the invention also relates to a chemolithoautotrophic bacterium of the genus *Hydrogenovibrio* that has been genetically modified to overexpress one or more endogenous or exogenous ectoine biosynthesis genes. The invention also relates to the use of such genetically modified bacterium for the production of ectoine.

## Description

The present invention generally relates to the field of producing ectoine in a bacterial organism. In particular, the invention relates to a method that consumes carbon dioxide and comprises culturing a chemolithoautotrophic bacterium which belongs to the genus *Hydrogenovibrio* under suitable conditions to produce ectoine. The ectoine produced by the chemolithoautotrophic bacterium according to the method of the present invention is secreted into the culture medium and can be readily obtained therefrom. In another aspect, the invention also relates to a chemolithoautotrophic bacterium of the genus *Hydrogenovibrio* that has been genetically modified to overexpress one or more endogenous or exogenous ectoine biosynthesis genes. The invention also relates to the use of such genetically modified bacterium for the production of ectoine.

### BACKGROUND OF THE INVENTION

It is widely recognized that human-induced carbon dioxide (CO₂) emission is the main cause of global warming. The German Federal Environment Agency estimated that industrial processes in Germany alone produce around 45 million tons of CO₂ emissions annually. To prevent the worst effects of global warming, international treaties were negotiated and adopted that sought to limit future emissions of greenhouse gases that contribute to global warming. Most significantly, the long-term temperature goal of the Paris climate agreement is to keep the rise in mean global temperature below 2 °C, and preferably below 1.5 °C above pre-industrial levels.

In the Intergovernmental Panel on Climate Change (IPCC) climate report of 2021, it is assumed that it will not be possible to reach the 1.5°C temperature goal without so-called "negative emissions", i.e. the active removal of CO₂ from the atmosphere. One simple way of removing CO₂ from the atmosphere resides in afforestation. According to studies, 3.6 billion tons of CO₂ per year could be bound during the growth phase, which corresponds to about 10 percent of the present CO₂ emissions. However, afforestation requires very large areas, which means that areas presently used for agriculture would have to be converted, thereby interfering with the ability to produce food for a growing world population. Storing CO₂ deep underground is another well-known possibility of achieving negative emissions. The CO₂ is first extracted from the ambient air by means of chemical processes and subsequently stored in underground geological formation, e.g. at the sea floor. This process is referred to as carbon capture and storage (CCS). Some CCS plants are already operating in Europe. While the CCS technology has great potential, it is presently extremely cost-intensive. The cost for removing a ton of CO₂ by CCS amounts to approximately 500-550 Euros. At present, the high costs are a barrier for using CCS in large scale.

Another idea that was discussed at a scientific level is the fertilization of the oceans with iron compounds. The rationale is to increase the nutrient content in the oceans such that plankton growth can be significantly increased which in turn leads to increased CO₂ binding. However, this idea is considered to involve a serious intervention into the ocean ecosystem which may be associated with severe and unpredictable side effects.

Another potential approach for providing negative emissions is the deployment of biotechnological processes that involve the consumption of CO₂. For example, it is known that certain microorganisms are able to convert CO₂ into organic carbon compounds. For example, it has been reported that autotrophic methanogenic archaea can consume CO₂ by converting it into methane. However, methane is another compound which contributes to global warming, and it does not seem to be possible to make products other than methane in these organisms. Another group of microorganisms that efficiently utilize CO₂ are photoautotrophic bacteria, and in particular cyanobacteria (Case & Atsumi, 2016). However, these organisms grow relatively slowly which is mainly due to the technically difficult input of light energy.

Yet another interesting approach for CO₂ utilization is already in the pilot stage. The 'Rheticus' project jointly carried out by Siemens and Evonik aims at the biotechnological production of butanol and hexanol by chemolithotrophic organisms (Hoff et al., 2021). The technology uses electricity from renewable energy for the conversion of CO₂ into CO and H₂. CO₂, CO, and H₂ are fed into a three-stage fermentation process designed by Evonik using *Clostridium autoethanogenum* and *Clostridium kluyveri* finally generating a 1:1 mixture of butanol and hexanol. This mixture can either be separated by distillation or be employed as a fuel mixture. It is estimated that within five years, an industrial plant using this technology can produce 10.000 tons of hexanol and butanol per year using 25,000 tons of CO₂ as starting material.

In light of the high amounts of CO₂ that are emitted in the industrial countries, there is a high need for additional biotechnological processes that involve the consumption of CO₂. These processes should preferably be feasible on an industrial scale so that as much CO₂ as possible is consumed. The processes should further be robust and easy to establish. In addition, the processes should result in the production of product that can be used, e.g. in medicine or cosmetics, or as food or feed additives. The present invention addresses this need and provides additional advantages as well.

### DESCRIPTION OF THE INVENTION

The present invention provides a new CO₂-consuming method for producing the compatible solute ectoine in a chemolithoautotrophic bacterium of the genus *Hydrogenovibrio.* The new method is amenable to large scale production and can be advantageously coupled to CO₂-producing methods. As a particular advantage, the method of the invention provides for the easy separation of ectoine from the cell culture.

Thus, in a first aspect, the present invention provides a method for producing ectoine, comprising the steps of
(a) providing a chemolithoautotrophic bacterium of the genus *Hydrogenovibrio;*
(b) culturing the bacterium in a culture medium that allows the propagation of the bacterium and contains at least 20 g/L NaCl;
(c) incubating the bacterium in a culture medium having a NaCl content of less than 20 g/L NaCl, and preferably less than 15 g/L NaCl;
(d) obtaining the ectoine from the culture medium; and
(e) optionally, obtaining the cells from the culture medium.

The method of the invention is directed to a production process of ectoine, a compound which is also known as 1,4,5,6-tetrahydro-2-methyl-4-pyrimidinecarboxylic acid. Ectoine is a small molecule amino acid derivate that is produced by some halophile and halotolerant microorganisms (Kunte et al. 2002). It serves as an osmolyte and is able to protect the producing cell against osmotic stress. It is thus referred to as a compatible solute. Three genes (*ectA, ectB* and *ectC*) have been identified for the synthesis of ectoine from L-aspartate *β*-semialdehyde (Louis & Galinski, 1997). Several microorganisms have been identified that contain genes necessary for ectoine biosynthesis and thus are expected to produce ectoine de novo (Cantera et al. 2022).

Ectoine has a broad range of application in the cosmetic, medical and biotechnological sector (Pastor et al., 2010). Industrially, ectoine is mainly produced by fermentation of the hetero-trophic microorganism *Halomonas elongata* in high-salt medium (Kunte et al., 2014). This process requires an organic carbon source and emits CO₂. In the first industrial fermentation process, a so called 'milking procedure' was used, in which ectoine is flushed out of the cells by osmotic down-shock. Besides natural producers of ectoine, common biotechnological host strains such as *E. coli* and *C*. *glutamicum* have been genetically modified and shown in lab-scale to produce ectoine in large quantities (Gießelmann et al., 2019; Wang et al., 2021). Such processes also require an organic carbon source and emit CO₂. Methanotrophic microorganisms have been demonstrated to produce ectoine from the greenhouse gas methane as carbon source, thus also emitting CO₂ (St 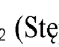 pniewska et al., 2014). Recently, it was also demonstrated that ectoine can be produced from CO₂ as sole carbon source, using the facultatively autotrophic bacterium *Halomonas stevensii* (Cantera et al., 2022). These strains, however, show very low biomass productivity, and as a consequence also a very low ectoine productivity. To increase ectoine production, these strains require the addition of glucose - and thus emit CO₂.

In step (a) of the method of the invention, a chemolithoautotrophic bacterium of the genus *Hydrogenovibrio* is provided. Bacteria belonging to this genus are able to utilize CO₂ as a carbon source and hydrogen (H₂) as an energy source. Since the final acceptor for the reducing equivalents generated by the respiratory chain can be oxygen (O₂), the expression host of the present invention is classified as an aerobic organism. Bacterial microorganisms of the genus *Hydrogenovibrio* have high CO₂ binding capacities which render them suitable for use in a method of the present invention. For example, wild-type *H. marinus* contains three different ribulose 1,5-bisphosphate carboxylase/oxygenases (RubisCOs) which allow the organism to adapt to different CO₂ levels in the environment (Toyoda et al., 2005; Toyoda et al., 2018). While known aerobic hydrogen-oxidizing bacteria are sensitive to high O₂ levels and grow comparably slowly, *H. marinus* displays a fast growth rate, even in the presence of high oxygen concentration of up to a pO₂ of 40%. Optimum growth was observed at a pO₂ between 5 and 10% (Nishihara et al. 1989). The complete genome of *H. marinus* has been sequenced (Arai et al., 2019; Jo et al., 2014).

It is particularly preferred according to the invention that the chemolithoautotrophic bacterium used in the process of the invention is of the species *Hydrogenovibrio marinus.* Suitable *Hydrogenovibrio marinus* strains for use in the method of the invention include *Hydrogenovibrio* marinus MH-110, which is deposited at the Japanese Cell Collection (JCM) under deposition number JCM 7688 and at the the Leibniz Institute DSMZ-German Collection of Microorganisms and Cell Cultures GmbH (DSMZ) under deposition number DSM 11271, and strains derived from the deposited strain.

As used herein, a bacterium is considered to belong to the species *H. marinus* if the 16S rRNA comprises:
(a) the nucleotide sequence of SEQ ID NO:1, or
(b) a nucleotide sequence having at least 99% or more sequence identity to the nucleotide sequences of SEQ ID NO:1.

Preferably, the nucleotide sequence has at least 99% or more sequence identity to the nucleotide sequences of SEQ ID NO:1 over a length of 400 nucleotides, 500 nucleotides, 600 nucleotides, 700 nucleotides, 800 nucleotides, 900 nucleotides, or 1000 nucleotides of the sequence of SEQ ID NO:1. More preferably, the nucleotide sequence has at least 99% or more sequence identity to the nucleotide sequences of SEQ ID NO:1 over the full length of the 1402 nucleotides. More preferably, the nucleotide sequence has at least 99.5% or more sequence identity to the nucleotide sequences of SEQ ID NO:1 over a length of 400 nucleotides, 500 nucleotides, 600 nucleotides, 700 nucleotides, 800 nucleotides, 900 nucleotides, or 1000 nucleotides of the sequence of SEQ ID NO:1. Even more preferably, the nucleotide sequence has at least 99.5% or more sequence identity to the nucleotide sequences of SEQ ID NO:1 over the full length of the 1402 nucleotides. More preferably, the nucleotide sequence has at least 99.8% or more sequence identity to the nucleotide sequences of SEQ ID NO:1 over a length of 400 nucleotides, 500 nucleotides, 600 nucleotides, 700 nucleotides, 800 nucleotides, 900 nucleotides, or 1000 nucleotides of the sequence of SEQ ID NO:1. Even more preferably, the nucleotide sequence has at least 99.8% or more sequence identity to the nucleotide sequences of SEQ ID NO:1 over the full length of the 1402 nucleotides.

*H. marinus* MH-110 was isolated from a seawater sample collected at the coast of Shonan in Japan. At the time of isolation, *H. marinus* MH-110 was the first known mesophilic, obligate chemolithoautotrophic and aerobic hydrogen-oxidizing bacterium. The optimal observed growth temperature is 37 °C. At 5 °C or 45 °C no growth is observed. As a marine bacterium, *H. marinus* grows optimally in an aqueous growth medium at about 0.5-0.6 M NaCl with a pH preference of around 6.5 (Nishihara et al., 1991b). In principle, seawater of a suitable salinity can be used to prepare growth media for potential biotechnological production processes employing *H. marinus* which has the advantage that the risk for microbial contamination is further reduced. Not many contaminating microorganisms are able to grow under chemolithoautotrophic conditions. Moreover, the use of seawater may be advantageous as it can be used in regions where freshwater is scarce.

The bacteria of the invention are chemolithoautotrophic which means that they obtain the energy required for their growth from the oxidation of inorganic compounds such as H₂. At the same time, they are autotrophic which means that they are able to produce complex organic compounds using an inorganic carbon source. In the case of bacteria from the genus *Hydrogenovibrio,* H₂ is used as an energy source and CO₂ as a carbon source. The particular advantage of *Hydrogenovibrio* is that due to its fast growth rate it sequesters relatively large amounts of CO₂ in relatively short time, rendering it an attractive host for CO₂-based climate-friendly industrial biotech processes. The ability of *Hydrogenovibrio* to adapt to different levels of CO₂ renders it a versatile host organism for CO₂-based biotech processes. In comparison to other chemolithotrophic or chemolithoautotrophic model organisms, e.g. *Cupriavidus necator, Hydrogenovibrio* is able to activate a carbon-concentrating mechanism that allows efficient carbon fixation under low-CO₂ conditions (Toyoda et al., 2018). Reducing the amount of CO₂ in the feed gas allows increasing the concentration of less soluble rate-limiting feed gasses like H₂ and O₂, thus further increasing the productivity of the industrial process. In step (b) of the method of the invention, the bacterium is incubated in a culture medium that allows the propagation of the bacterium and the production of ectoine. As used herein, a "culture medium" refers to any type of liquid medium that can be used for suspending bacterial cells. Suitable culture media for propagating *Hydrogenovibrio* bacteria have been described in the art (Nishihara et al., 1989; Arai & Ishii, 2019) and include conditions of temperature, pH and salt that allow for the growth and propagation of the cells. For *Hydrogenovibrio marinus,* an appropriate growth temperature will be in the range of 30-39 °C, whereas a growth temperature of 36-37 °C is particularly preferred. A pH in the range of 6.0-8.0 is preferred, more preferably 7.0-8.0, such as 6.5 (Nishihara et al., 1991b).

The culture medium used in step (b) contains at least 20 g/L NaCl, and preferably at least 25 g/L, at least 30 g/L, at least 35 g/L, at least 40 g/L, at least 45 g/L, at least 50 g/L, at least 55 g/L, at least 60 g/L, at least 65 g/L, at least 70 g/L NaCl, at least 80 g/L NaCl or more. It has been found herein that the use of culture media comprising less than 20 g/L NaCl do not allow the production of significant amounts of ectoine. In the presence of 20 g/L NaCl or more, the bacteria will automatically produce ectoine.

Cell culturing is preferably performed in a gaseous atmosphere containing H₂, CO₂ and O₂. A wide variety of different ratios of the three gases can be used for generating the atmosphere used for culturing. Nitrogen (N₂), other inert gases, or other gasses that are not consumed by *H. marinus,* can be present in various amounts to generate the target ratio of H₂, CO₂ and O₂. Thus, in one embodiment, the gas atmosphere for culturing the bacterium in comprises H₂, CO₂, O₂ and N₂.

It must be noted that atmosphere compositions which contain 4% or more O₂ and, at the same time, 4% or more H₂, are explosive which means that special security measures must be taken during culturing. For lab scale experiments, atmosphere compositions containing less than 4% O₂ and, at the same time, less than 4% H₂ may be used to avoid the risk of explosions. However, working with these concentrations of H₂ and O₂ has been found to lower the ectoine yield. Therefore, when seeking to achieve optimum amounts of ectoine, it is preferred that both O₂ and H₂ are used in an amount of 4% or more when producing the atmosphere compositions for cell culturing under production conditions.

All percentages recited herein in connection with the composition of the atmosphere for cell culturing refer volume percent (v/v) based on the overall volume of the atmosphere.

In one embodiment, H₂ is present in the atmosphere composition for cell culturing in an amount of 80-89%, more preferably 85-89%, and even more preferably 87-89%. In such embodiment, the CO₂ can be present in the atmosphere composition in an amount of 0.1-10%, preferably 2-10%, more preferably 5-10%, and even more preferably 8-10%. In such embodiment, the O₂ is present in the atmosphere composition in an amount of 0.5-3%, more preferably 1-3%, and even more preferably 2-3 %. The atmosphere composition may also include N₂ in an amount of up to 2%. Preferred atmosphere compositions include H₂:CO₂:O₂:N₂ = 89:10:1:0, 88:10:2:0, and 87:10:3:0. Alternative preferred atmosphere compositions include H₂:CO₂:O₂:N₂ = 82:15:3:0, 83:15:2:0, and 84:15:1:0. Yet other alternative preferred atmosphere compositions include 98:1:1:0, 97:1:2:0, and 97:1:3:0.

In another embodiment, H₂ is present in the atmosphere composition for cell culturing in an amount of 70-79%, more preferably 75-79%, and even more preferably 77-79%. In such embodiment, the CO₂ is present in the atmosphere composition in an amount of 0.1-10%, more preferably 5-10%, and even more preferably 8-10 %. In such embodiment, the O₂ can be present in the atmosphere composition in an amount of 0.1-10%, preferably 2-10%, more preferably 5-10%, and even more preferably 8-10%. The atmosphere composition may also include N₂ in an amount of up to 5%. Preferred atmosphere compositions include H₂:CO₂:O₂:N₂ = 77:10:10:3, 78:10:10:2, and 79:10:10:1.

In yet another embodiment, H₂ is present in the atmosphere composition for cell culturing in an amount of 0.5-3%, more preferably 1-3%, and even more preferably 2-3%. In such embodiment, the CO₂ is present in the atmosphere composition in an amount of 0.1-10%, more preferably 5-10%, and even more preferably 8-10 %. In such embodiment, the O₂ is present in the atmosphere composition in an amount of 2-10%, more preferably 5-10%, and even more preferably 8-10%. The atmosphere composition may also include N₂ in an amount of up to 80%. Preferred atmosphere compositions include H₂:CO₂:O₂:N₂ = 3:9:8:80, 3:8:9:80, 3:9:9:79, and 2:10:10:78.

For production conditions which seek to achieve optimum amounts of ectoine, the conditions in the atmosphere composition will be selected such that both H₂ and O₂ are present in an amount of 4% or more, preferably 10% or more, such as 15% or more, 20% or more, 25% or more, 30% or more, 35% or more, or 40% or more. For example, the H₂ is present in the atmosphere composition for cell culturing in an amount of 50-95%, the O₂ is present in an amount of 4-40%, and the CO₂ is present in an amount of 0.1-10%. N₂ may or may not be present. Exemplary atmosphere compositions comprise the following:
H₂:CO₂:O₂ = 60:2:38;
H₂:CO₂:O₂ = 62:2:36;
H₂:CO₂:O₂ = 64:2:34;
H₂:CO₂:O₂ = 66:2:32;
H₂:CO₂:O₂ = 68:2:30;
H₂:CO₂:O₂ = 70:2:28;
H₂:CO₂:O₂ = 72:2:26;
H₂:CO₂:O₂ = 74:2:24;
H₂:CO₂:O₂ = 76:2:22;
H₂:CO₂:O₂ = 78:2:20;
H₂:CO₂:O₂ = 80:2:18;
H₂:CO₂:O₂ = 82:2:16;
H₂:CO₂:O₂ = 84:2:14;
H₂:CO₂:O₂ = 86:2:12;
H₂:CO₂:O₂ = 88:2:10;
H₂:CO₂:O₂ = 90:2:8;
H₂:CO₂:O₂ = 92:2:6;
H₂:CO₂:O₂ = 94:2:4.
H₂:CO₂:O₂ = 60:1:39;
H₂:CO₂:O₂ = 62:1:37;
H₂:CO₂:O₂ = 64:1:35;
H₂:CO₂:O₂ = 66:1:33;
H₂:CO₂:O₂ = 68:1:31;
H₂:CO₂:O₂ = 70:1:29;
H₂:CO₂:O₂ = 72:1:27;
H₂:CO₂:O₂ = 74:1:25;
H₂:CO₂:O₂ = 76:1:23;
H₂:CO₂:O₂ = 78:1:21;
H₂:CO₂:O₂ = 80:1:19;
H₂:CO₂:O₂ = 82:1:17
H₂:CO₂:O₂ = 84:1:15
H₂:CO₂:O₂ = 86:1:13
H₂:CO₂:O₂ = 88:1:11
H₂:CO₂:O₂ = 90:1:9
H₂:CO₂:O₂ = 92:1:7
H₂:CO₂:O₂ = 94:1:5

Conveniently, it is also possible to culture the *Hydrogenovibrio* strains of the invention in a gaseous atmosphere that comprises ambient air (which includes about 78% N₂, about 21% O₂ and some other gases) that has been supplemented with 0.1-10% CO₂ and 1-3% H₂. Such atmospheres are not explosive and remain not explosive even when getting in contact with ambient air. In one preferred embodiment, the gaseous atmosphere comprises ambient air that has been supplemented with 10% CO₂ and 3% H₂ or ambient air that has been supplemented with 10% CO₂ and 2% H₂. In another preferred embodiment, the gaseous atmosphere comprises ambient air that has been supplemented with up to 50%, 60%, 70, or 80% H₂, and supplemented with 0.1% - 10% CO₂, such as 0.1%, 1%, 5% or 10% CO₂.

The *Hydrogenovibrio* strain of the invention is cultured until a suitable optical density (OD) has been reached. Once a sufficiently high OD has been reached, the culturing is stopped and the ectoine is harvested. The OD that is to be reached will depend on the overall volume of the culture. If a lab-scale fermenter with a volume of 1-5 liter is used, the ectoine is preferably harvested after the culture has reached an OD that corresponds to an OD at wavelength 540 nm (OD₅₄₀) of 0.5 to 2.0, more preferably an OD₅₄₀ of 0.75 to 1.5, and even preferably an OD₅₄₀ of 1.0 to 1.2. If an industrial fermenter with a volume of 100 liter or more is used, the ectoine is preferably harvested after the culture has reached an OD₅₄₀ of 5.0 to 50.0, more preferably an OD₅₄₀ of 10.0 to 40.0, and even preferably an OD₅₄₀ of 15.0 to 25.0.

As shown in the below Example 1, when using a wild-type *Hydrogenovibrio* strain like MH-110, most of the ectoine produced by the cell accumulates within the cell. About 95% of the total ectoine is found in the cell lysate, whereas only about 5% is released into the surrounding media. The process of the invention has the particular advantage that it includes an osmotic down-shock by which the ectoine is flushed out of the cells. In this way, the ectoine can be easily obtained by purifying it from the culture supernatant. Hence, after culturing the bacterium in step (b) of the above method, the bacterium is incubated in step (c) in a culture medium having a NaCl content of less than 20 g/L NaCl, and preferably less than15 g/L NaCl. The medium used for the osmotic down-shock can be the same as the medium used in step (b) or it can be a different medium, such as a solution of NaCl in H₂O. In one preferred embodiment, the media used in steps (b) and (c) of the method are identical except for the NaCl content. In another preferred embodiment, the medium used in step (c) is water or NaCl solved in water. The transfer into the new medium can be effected in any way that is commonly used in the field of bacterial cell culturing. For example, the cells can be harvested after step (b) and transferred into a new culture vessel containing the low-salt medium. Alternatively, where the culturing technique is based on a continuous process, one may gradually exchange the high-salt medium by the low-salt medium. By transferring the bacterium into conditions of low NaCl, the ectoine is flushed out of the cells and accumulates in the surrounding culture medium.

The medium used for the osmotic down-shock has a NaCl content of below 20 g/L NaCl, and preferably 19 g/L NaCl or less, 18 g/L NaCl or less, 17 g/L NaCl or less, 16 g/L NaCl or less, 15 g/L NaCl or less, 14 g/L NaCl or less, 13 g/L NaCl or less, 12 g/L NaCl or less, 11 g/L NaCl or less, 10 g/L NaCl or less, 9 g/L NaCl or less, 8 g/L NaCl or less, 7 g/L NaCl or less, 6 g/L NaCl or less, and more preferably 5 g/L NaCl or less.

In step (d) of the method, the ectoine is obtained from the culture medium. The harvesting step can be performed by routine processes. For example, the ectoine can be purified from protein and other components by standard methods that have been described in the art and may comprise, e.g. filtration, desalination, cation exchange, and extraction of crude ectoine (Chen et al. 2017).

Since the cells are not significantly disrupted during the osmotic down-shock, the culturing of the cells under normal-salt or high-salt conditions can be continued after the osmotic down-shock such that the cells produce new ectoine which accumulates into the cells and can be flushed out again by repeating the down-shock. In a preferred embodiment, the cells are subjected to more than one osmotic down-shock during the culturing period. In practice this means that steps (b) to (d) of the above methods are repeated several times, such as 2 times, 3 times, 4 times, or 5 times during the production process.

In one preferred embodiment, the method of the invention is directed to the simultaneous production of ectoine and bacterial biomass. For this purpose, not only the ectoine, but also the bacterial cells are harvested. Such method hence also comprises a step (e) which comprises obtaining the cells from the culture medium. The whole bacterial biomass can be harvested without disruption of the cells, e.g., by centrifugation and/or filtration. Subsequently, the harvested cells can be dried, preferably by spray-drying or freeze-drying. The powder containing the biomass can then be used as an additive for food or feed. Preferably, the powder containing the biomass is used as an additive for animal feed, more preferably fish or shrimp feed.

In another preferred embodiment, the proteins contained in the bacterial cells are enriched or isolated. For this purpose, the method of the invention may further comprise a step (f) which relates to obtaining the proteins from the bacterial cells. Normally, such step includes the lysis of the cells, as described elsewhere herein, and the subsequent harvest of the proteins by routine methods. Typically, the cells are first disrupted, e.g., by enzymatic lysis, e.g. using lyso-zyme, by chemical lysis, e.g. using detergents, NaOH, or the like, or by mechanical means, e.g. using a French press or bead mill. Cell lysis can also be achieved by repeating cycles of freezing and thawing or by sonication. Cellular debris can be easily removed from the protein fraction by centrifugation. The protein can then be purified from other components by standard methods that have been described in the art and may comprise, e.g. gel filtration, ion exchange chromatography (IEX), affinity chromatography, hydrophobic interaction chromatography (HIC) or other methods which have been described in the context of protein purification.

It is a particular advantage of the method that the ectoine and the protein-rich bacterial biomass are harvested in separate steps. The ectoine is flushed out of the cells and does not accumulate within the cells. At the same time, the protein remains in the cells and may be extracted after cell lysis. Accordingly, the different products need not to be harvested together and then separated, which simplifies the purification of each of these products.

In one embodiment, the method of the invention uses a naturally occurring *Hydrogenovibrio* strain, preferably a *Hydrogenovibrio marinus* strain, and more preferably *Hydrogenovibrio marinus* strain MH-110, which has not been genetically modified. In another embodiment, the method of the invention uses a *Hydrogenovibrio* strain, preferably a *Hydrogenovibrio marinus* strain, and more preferably a *Hydrogenovibrio marinus* strain MH-110, which has been genetically modified. As used herein, "genetically modified" means that either the naturally occurring genome of the strain has been altered by recombinant means, e.g. by incorporating or deleting nucleic acid sequences into or from the genome, or extrachromosomal elements have been introduced a strain, e.g. plasmids or integrative genetic elements that code for biosynthesis genes, such as the ectoine biosynthesis genes.

The *Hydrogenovibrio* strain used in the above method has been genetically modified to overexpress one or more endogenous or exogenous ectoine biosynthesis genes. As used herein, "ectoine biosynthesis genes" include the genes *ectA, ectB* and *ectC* which encode the enzymes L-2,4-diaminobutyric acid acetyltransferase, L-2,4-diaminobutyric acid transaminase and L-ectoine synthase, respectively (Louis & Galinski, 1997). Accordingly, the bacterium used in the process of the invention has been genetically modified to overexpress one or more of the endogenous or exogenous biosynthesis genes *ectA, ectB,* and *ectC.*

In one embodiment, one or more of the biosynthesis genes which are expressed are endogenous genes, i.e. the genes are derived from the *Hydrogenovibrio* species that is used in the method of the invention. In such embodiments, the strain used for ectoine production is genetically modified to include one or more additional copies of the endogenous biosynthesis gene or genes, e.g. as part of a plasmid or expression vector, or integrated into the genome. Due to the additional copies, the ectoine production can be effectively increased. In such embodiments, the term "overexpression" refers to the fact that the one or more ectoine biosynthesis genes are expressed at increased levels relative to the corresponding wild-type that comprises only a single copy of said genes, thereby leading to higher amounts of the encoded enzyme compared to the wild-type. In a particularly preferred embodiment, the bacterium used for ectoine production has been genetically modified to overexpress the endogenous biosynthesis genes *ectA* (SEQ ID NO:29), *ectB* (SEQ ID NO:30) and *ectC* (SEQ ID NO:31). In such an embodiment, the entire ectoine biosynthesis gene cluster of *H. marinus* is overexpressed. In yet another particularly preferred embodiment, the bacterium used for ectoine production has been genetically modified to overexpress only the endogenous gene *ectB* (SEQ ID NO:30), whereas none of the other endogenous biosynthesis genes *ectA* (SEQ ID NO:29) and *ectC* (SEQ ID NO:31) is overexpressed as a result of the genetic modification. In such embodiments, the bacterium has been genetically modified such that only the biosynthesis gene *ectB* is overexpressed.

In another embodiment, the one or more of the biosynthesis genes which are expressed are exogenous genes, i.e. the genes are derived from a *Hydrogenovibrio* species other than *Hydrogenovibrio marinus* or from a bacterial genus other than *Hydrogenovibrio.* In such embodiments, the bacterium used for ectoine production is genetically modified to include one or more copies the exogenous biosynthesis gene(s), e.g. as part of a plasmid or expression vector. Due to the exogenous gene or genes, the ectoine production can be effectively increased. In such embodiments, the term "overexpression" refers to the fact that the one or more ectoine biosynthesis genes are expressed at increased levels relative to the corresponding wild-type that does not comprise and express the gene. In this way, the amounts of the encoded enzymes are higher in the modified bacterium compared to the wild-type. In a particularly preferred embodiment, the bacterium used for ectoine production has been genetically modified to overexpress one or more biosynthesis genes from *Halomonas elongata,* e.g., the strain deposited under DSM 2581, or from *Pseudomonas stutzeri* e.g., the strain deposited under DSM 4166. In a particularly preferred embodiment, the bacterium used for ectoine production has been genetically modified to overexpress exogenous biosynthesis genes *ectA, ectB* and *ectC.* In such an embodiment, the entire ectoine biosynthesis gene cluster of the foreign organism is overexpressed. In yet another particularly preferred embodiment, the bacterium used for ectoine production has been genetically modified to overexpress an exogenous gene *ectB* of an ectoine-producing bacterium, whereas no exogenous *ectA* and *ectC* gene is overexpressed. In such embodiments, the bacterium has been genetically modified such that only exogenous *ectB* is overexpressed.

For example, In a particularly preferred embodiment, the bacterium used for ectoine production has been genetically modified to overexpress the biosynthesis genes *ectA, ectB* and *ectC* from the ectoine operon of *P. stutzeri* (SEQ ID NO:32). In a particularly preferred embodiment, the bacterium used for ectoine production has been genetically modified to overexpress the biosynthesis genes *ectA* (SEQ ID NO:33), *ectB* (SEQ ID NO:34) and *ectC* (SEQ ID NO:35) of *P. stutzeri.* In yet another particularly preferred embodiment, the bacterium used for ectoine production has been genetically modified to overexpress the gene *ectB* of *P. stutzeri* (SEQ ID NO:34), whereas no exogenous *ectA* (SEQ ID NO:33) and *ectC* gene (SEQ ID NO:35) is overexpressed. In such embodiments, the bacterium has been genetically modified such that only exogenous *ectB* is overexpressed. Preferably, the exogenous *ectB* gene expressed is derived from the *P. stutzeri* strain deposited at the the Leibniz Institute DSMZ-German Collection of Microorganisms and Cell Cultures GmbH (DSMZ) under deposition number DSM 4166.

In one embodiment, the genetic modification is effected by introduction of a plasmid. As used herein, a plasmid refers to an extrachromosomal circular DNA capable of autonomous replication in a cell. In one embodiment the plasmid is an expression vector that comprises a nucleotide sequence that encodes the protein or proteins to be produced by the cell. Expression vectors are typically DNA constructs that are optimized for protein expression and comprise an origin of replication that allows the vector to replicate independently of the cell. Expression vectors also include regulatory control elements that are functionally linked to the nucleotide sequences to be expressed and control their transcription and/or translation. Suitable control elements include constitutive or controllable prokaryotic promoters, a transcription termination sequence and a ribosome binding site (RBS). Expression vectors may also include one or more antibiotic resistance genes which allow transformed cells to be selected by use of media that contain antibiotics.

Suitable plasmids for use in a *Hydrogenovibrio* strain, such as a *H. marinus* strain, have been described in applicant's application WO 2023/031444 A1 and include the plasmids pHM38, pHM39 and pHM40 which are provided herein as SEQ ID NO:2, SEQ ID NO:3 and SEQ ID NO:4, respectively. These plasmids are based on the pAM5409 backbone (Bishé, et al., 2019). Plasmid pHM38 has been generated by cloning a gene encoding the green fluorescent protein (GFP) into the pAM5409 plasmid backbone. Plasmid pHM39 is based on pHM38 and differs from the latter by substitution of the J23119 promoter and the BBa_B0034 ribosome binding site with the J23111 promoter and the BBa_B0064 ribosome binding site. Plasmid pHM40 is based on pHM38 and differs from the latter by substitution of the J23119 promoter and the BBa_B0034 ribosome binding site with the J23114 promoter and the BBa_B0031 ribosome binding site. Further used in plasmid pHM42 which is provided herein as SEQ ID NO:5 and does not include an insert.

The plasmid or expression vector comprised by the chemolithoautotrophic bacterium used in the method of the invention preferably comprises a synthetic promoter, more preferably a promoter selected from the group of J23119 (SEQ ID NO:6), J23111 (SEQ ID NO:7) and J23114 (SEQ ID NO:8). The plasmid or expression vector comprised by the chemolithoautotrophic bacterium used in the method of the invention preferably also comprises a synthetic ribosomal binding site, more preferably a ribosomal binding site selected from the group of BBa_B0034 (SEQ ID NO:9), BBa_B0064 (SEQ ID NO:10), and BBa_B0031 (SEQ ID NO:11).

In one embodiment, the plasmid or expression vector comprised by the modified chemolithoautotrophic bacterium used in the method of the invention comprises the naturally occurring promoter of the *H. marinus* ectoine operon, *Pect* (SEQ ID NO:27). In another embodiment, the plasmid or expression vector comprised by the chemolithoautotrophic bacterium used in the method of the invention comprises the naturally occurring RBS of the *H. marinus* ectoine operon. In yet another embodiment, the plasmid or expression vector comprised by the chemolithoautotrophic bacterium used in the method of the invention comprises both the naturally occurring promoter (SEQ ID NO:27) and the naturally occurring RBS of the *H. marinus* ectoine operon (SEQ ID NO:28).

The plasmid or expression vector can be cloned in accordance with routine methods known in the art which are described, e.g. in Maniatis et al. 1982, Molecular Cloning, A laboratory Manual, Cold Spring Harbor Laboratory. The plasmid or expression vector is preferably introduced into the chemolithoautotrophic bacterium of the invention by parental mating with a suitable donor strain. For this purpose, the plasmid or expression vector is first introduced into an *E. coli* strain, such as *E. coli* WM3064, by routine methods, such as electroporation or calcium phosphate- or rubidium chloride-mediated transformation. In a subsequent step, the plasmid or expression vector is transferred from the donor strain WM3064 to the *Hydrogenovibrio* strain by spreading and incubating aliquots from cultures of both strains on a nitrocellulose filter. In addition, an electroporation process for introducing a plasmid or an expression vector into a chemolithoautotrophic bacterium, preferably into a *Hydrogenovibrio* strain, such as a *H. marinus* strain, has been developed. Both procedures are described in applicant's application WO 2023/031444 A1.

In another preferred embodiment, the *Hydrogenovibrio* strain used in one of the above methods has been modified to comprise an integrative genetic element, i.e. an element that stably integrates into the genome. A method for integration of DNA into the genome of *H. marinus* has been developed and described in applicant's application WO 2023/031444 A1, see Example 2. For example, the expression cassettes described above, containing the ectoine biosynthesis genes with their respective promoter and an antibiotic (e.g. kanamycin) resistance gene, can be flanked with DNA-fragments complementary to the genomic region of the endogenous bla-locus. The DNA-fragments can then be transferred to *H. marinus* via electroporation. Successful integrations can then be selected for the respective antibiotic. The an integrative genetic element can comprise any of the above-mentioned promoters or ribosome binding sites.

In one preferred embodiment, the *Hydrogenovibrio* bacterium of the present invention has been genetically modified such that the ectoine production is at least 1.5-fold, at least 2-fold, at least 2.5-fold, at least 3-fold, at least 3.5-fold, at least 4-fold, at least 4.5-fold, at least 5-fold, or more increased relative to ectoine production in the corresponding wild-type strain, e.g. *Hydrogenovibrio marinus* strain MH-110.

In the event that a genetically modified *Hydrogenovibrio* bacterium is used for the production of ectoine, it is preferred that at least 2% (w/w) of the biomass produced by the *Hydrogenovibrio* strain used in the method of the invention is ectoine. More preferably, at least 3% (w/w), at least 4% (w/w), at least 5% (w/w), at least 6% (w/w), at least 7% (w/w), at least 8% (w/w), at least 9% (w/w), at least 10% (w/w), at least 11% (w/w), at least 12% (w/w), at least 13% (w/w), at least 14% (w/w) or at least 15% (w/w) of the biomass produced by the *Hydrogenovibrio* strain used in the method of the invention is ectoine.

Apart from ectoine, the genetically modified *Hydrogenovibrio* strain used in the method of the invention may also produce proteins or other compounds that are of commercial value. For example, the *Hydrogenovibrio* strain used in the above method may have been further modified to produce a therapeutically active protein, i.e. a protein that can be used in human or veterinary medicine. The type of therapeutically active protein is not particularly limited and can include cytokines, hormones, growth factors, and the like. In one embodiment, the *Hydrogenovibrio* strain used in the method of the invention produces an interferon protein. The type of interferon is not particularly limited. Generally, the interferon proteins selected for expression in the host cells of the invention can be of human or non-human origin. The interferon protein can belong to any interferon subclass that is known in the art. In particular, the interferon protein to be expressed in the host cell of the invention can be an alpha-interferon (IFN-α), a beta-interferon (IFN-β) or a gamma-interferon (IFN-γ). In a particularly preferred embodiment, the interferon protein to be expressed is human IFN-α, IFN-β or IFN-γ or a biologically active fragment thereof. In another embodiment, the *Hydrogenovibrio* strain used in the method of the invention may have been further modified to produce human somatotropin or a biologically active fragment thereof. Human somatotropin, which is a protein that is also referred to in the literature as human growth hormone (hGH), is a protein that is produced by the pituitary gland in the brain and stimulates the liver to produce somatomedins, i.e. hormones that are very similar in structure to insulin and stimulate the growth of bones. The production of human somatotropin is discussed in detail in applicant's application WO 2023/031444 A1.

Any of the above ectoine-producing methods of the invention can be advantageously coupled with processes that produce CO₂. For example, the ectoine-producing methods of the invention can be coupled with other biotechnological processes in the sense that the CO₂ which is generated during the other biotechnological processes is used in the ectoine-producing methods of the invention. In one preferred embodiment, the culturing step (b) of the above methods of the invention uses CO₂ gas derived from an aquaculture. CO₂-containing water that is produced during the aquaculture process is processed to separate the CO₂ from the water, and the CO₂ is subsequently used as a carbon source for the bacteria used in the ectoine-producing method of the invention. In this way, a closed CO₂ circuit can be established, as described in WO 2021/220056 A1.

According to the invention, the *Hydrogenovibrio* strain used in one of the above methods may produce, apart from ectoine, also one or more heterologous proteins. As used herein, a heterologous protein is a protein which is not encoded by a gene of the non-modified organism, i.e. the naturally occurring *Hydrogenovibrio* strain, and therefore not produced by the non-modified organism. The overproduction and extraction of a heterologous protein in cells of the genus *Hydrogenovibrio* has been described in applicant's application WO 2023/031444 A1. Preferably, the chemolithoautotrophic bacterium of the present invention has been genetically modified to produce a mammalian, more preferably a human protein. The protein preferably is a therapeutically active protein, i.e. a protein that can be used in human or veterinary medicine.

In a second aspect, the invention relates to a chemolithoautotrophic bacterium as described above, i.e. a bacterium of the genus *Hydrogenovibrio* that has been genetically modified to overexpress one or more endogenous or exogenous ectoine biosynthesis genes. The chemolithoautotrophic bacterium preferably is of the species *Hydrogenovibrio marinus.* More preferably, the chemolithoautotrophic bacterium is or is derived from *Hydrogenovibrio* marinus MH-110, deposited at the Japanese Cell Collection (JCM) under deposition number JCM 7688, or at the the Leibniz Institute DSMZ-German Collection of Microorganisms and Cell Cultures GmbH (DSMZ) under deposition number DSM 11271. Preferably, the bacterium has been genetically modified to overexpress one or more endogenous or exogenous biosynthesis genes selected from the group comprising *ectA, ectB,* and *ectC,* as described above. It is particularly preferred, as described above, that the bacterium has been genetically modified to overexpress the endogenous or exogenous biosynthesis gene *ectB,* but has not been genetically modified to overexpress the endogenous or exogenous biosynthesis genes *ectA* and *ectC.*

In a third aspect, the invention relates to the use of the chemolithoautotrophic bacterium of the genus *Hydrogenovibrio* for the production of ectoine. The chemolithoautotrophic bacterium has been described above and preferably is of the species *Hydrogenovibrio marinus.* More preferably, the chemolithoautotrophic bacterium is or is derived from *Hydrogenovibrio* marinus MH-110, deposited at the Japanese Cell Collection (JCM) under deposition number JCM 7688, or at the the Leibniz Institute DSMZ-German Collection of Microorganisms and Cell Cultures GmbH (DSMZ) under deposition number DSM 11271. Preferably, the bacterium has been genetically modified to overexpress one or more endogenous or exogenous biosynthesis genes selected from the group comprising *ectA, ectB,* and *ectC,* as described above. It is particularly preferred, as described above, that the bacterium has been genetically modified to overexpress the endogenous or exogenous biosynthesis gene *ectB,* but has not been genetically modified to overexpress the endogenous or exogenous biosynthesis genes *ectA* and *ectC.*

Finally, in a fourth aspect, the invention relates to a method for producing ectoine by use of a genetically modified microorganism, comprising the steps of
(a) providing a chemolithoautotrophic bacterium of the genus *Hydrogenovibrio* as described herein above which has been genetically modified to overexpress one or more endogenous or exogenous ectoine biosynthesis genes;
(b) culturing the bacterium in a culture medium that allows the propagation of the bacterium and contains at least 20 g/L NaCl;
(c) obtaining the ectoine from the culture medium and/or the cells.

The method of the fourth aspect of the invention is directed to a production process of ectoine which uses a genetically modified bacterium as described herein. In step (a) of the method, a chemolithoautotrophic bacterium of the genus *Hydrogenovibrio* is provided which has been genetically modified to overexpress one or more endogenous or exogenous ectoine biosynthesis genes. The bacterium has been modified to overexpress one or more endogenous or exogenous ectoine biosynthesis genes. The endogenous or exogenous ectoine biosynthesis genes which are overexpressed have been described in detail above. Preferably, the one or more endogenous or exogenous ectoine biosynthesis genes are selected from the group consisting of genes *ectA, ectB,* and *ectC.* Alternatively, the bacterium can be genetically modified to overexpress the endogenous or exogenous biosynthesis gene *ectB,* but has not been genetically modified to overexpress the endogenous or exogenous biosynthesis genes *ectA* and *ectC.*

The culturing step (b) in the presence of at least 20 g/L NaCl can be performed as described in more detail above. Suitable culture media and culturing conditions, e.g., gas atmospheres have been described herein above. The method of the fourth aspect of the invention may or may not comprise an osmotic down-shock step as described above in which the bacterium is contacted with a medium that contains a NaCl content of less than 20 g/L NaCl, and preferably less than 15 g/L NaCl to flush out the produced ectoine.

In case no osmotic down-shock is performed, the ectoine will accumulate and can be obtained from the cells after cell lysis as described above. On the other hand, if the method includes an osmotic down-shock step, the ectoine can be obtained from the culture supernatant according to routine purification methods.

### BRIEF DESCRIPTION OF THE FIGURES

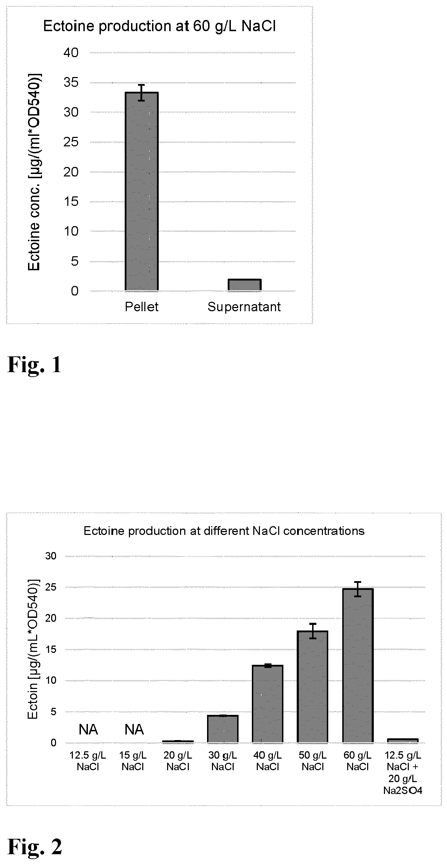

**Figure 1** shows the results of ectoine quantification of *H. marinus* MH-110 culture samples grown in media containing 60 g/L NaCl. Ectoine was measured in the cell lysates and in the culture supernatants. Error bar of cell lysate indicates standard deviation of n=2 technical replicates. Supernatant was measured as n=1.
**Figure 2** shows the result of ectoine quantification of *H. marinus* MH-110 culture samples grown in Basal media with NaCl concentration ranging from 12.5 to 60 g/L. One culture containing 12.5 g/L NaCl was supplemented with 20 g/L Na₂SO₄. Ectoine was calculated as the sum of ectoine found in the cell pellets and in the supernatant of the cultures. For data marked with "NA", ectoine was below detection limit. Error bars indicate the standard deviation of n=3 technical replicates.

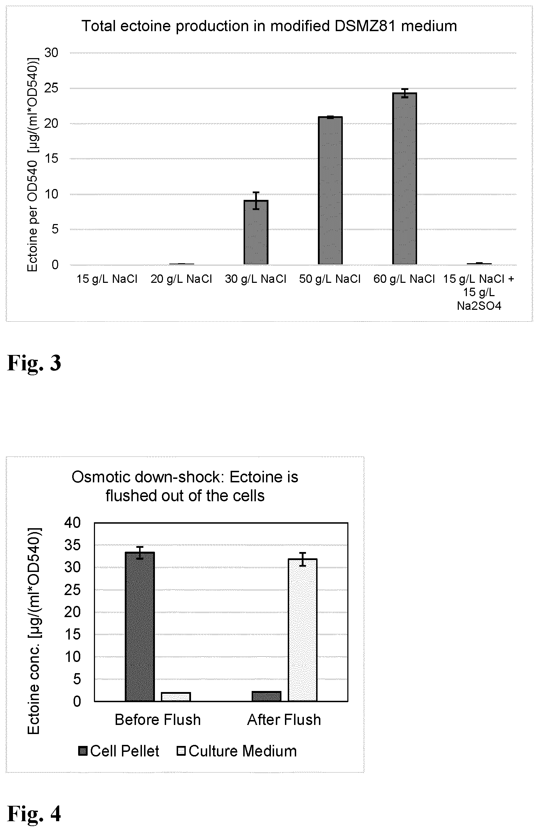

**Figure 3** shows the result of ectoine quantification of *H. marinus* MH-110 culture samples grown in modified DSMZ81 media with NaCl concentration ranging from 12.5 to 60 g/L, and in media containing 15 g/L NaCl and 15 g/L Na₂SO₄. Total ectoine was calculated as the sum of ectoine measured in the cell pellet and supernatant of each culture. For data marked with "NA", ectoine was below detection limit. Error bars indicate the standard deviation of n=3 technical replicates
**Figure 4** shows the result of ectoine quantification of *H. marinus* MH-110 culture samples. Ectoine was quantified in cells lysates ("Cell Pellet") or in culture supernatant ("Culture Medium") before and after the osmotic down-shock ("flush") step using low-salt medium containing 15 g/L NaCl. Error bars indicate the standard deviation of n=2 technical replicates.

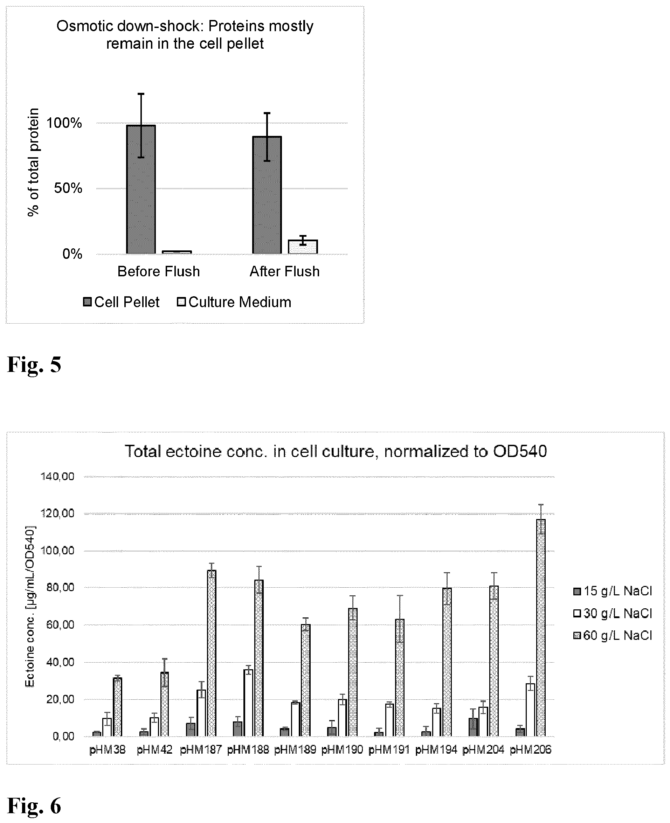

**Figure 5** shows the amount of the proteins of *H. marinus* MH-110 culture samples intracellularly and in the culture supernatant before and after the osmotic down-shock ("flush") using low-salt medium containing 15 g/L NaCl. Error bars indicate standard deviation of n=3 technical replicates.
**Figure 6** shows the total amount of ectoine produced in the genetically modified *H. marinus* strains. The recombinant strains pHM187 - pHM206 carry replicative plasmids with genes of the ectoine biosynthesis operon. The recombinant strain pHM38 is a modified *H. marinus* strain that expresses GFP. The recombinant strain pHM42 is a modified *H. marinus* strain that carries an empty plasmid. The recombinant strains pHM38 and pHM42 serve as controls, as they contain only the native endogenous ectoine biosynthesis genes. Ectoine was measured in the cell pellets and in the culture supernatants and combined to determine total ectoine production. Error bars show standard deviation of n=3 technical replicates.

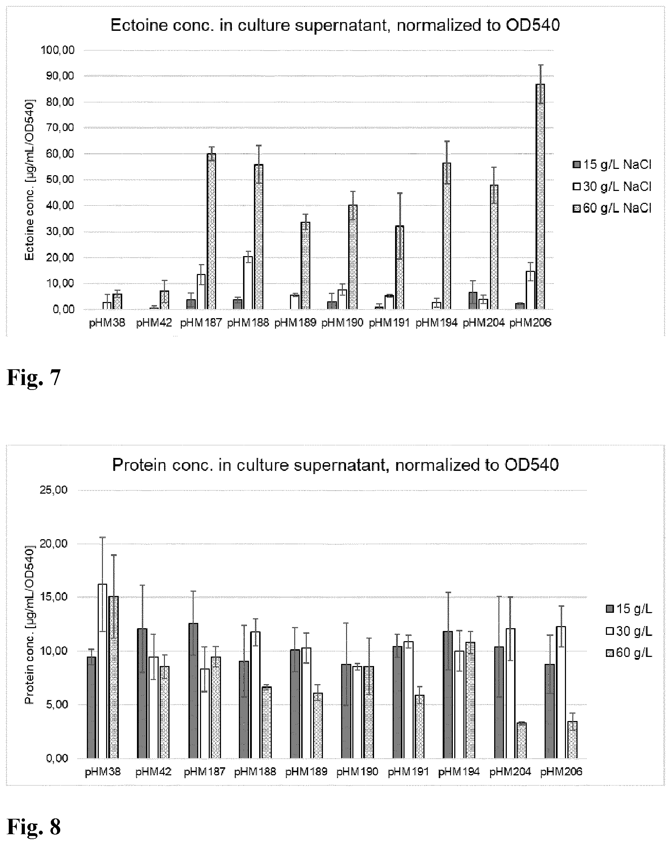

**Figure 7** shows the amount of ectoine in the culture supernatant of the genetically modified *H. marinus* strains. The recombinant strains pHM187 - pHM206 carry replicative plasmids with genes of the ectoine biosynthesis operon. The recombinant strain pHM38 is a modified *H. marinus* strain that expresses GFP. The recombinant strain pHM42 is a modified *H. marinus* strain that carries an empty plasmid. The recombinant strains pHM38 and pHM42 serve as controls, as they contain only native ectoine biosynthesis genes. Error bars show standard deviation of n=3 technical replicates.
**Figure 8** shows the total protein concentration in the culture supernatants of genetically modified *H. marinus* strains. The recombinant strains pHM187 - pHM206 carry replicative plasmids with genes of the ectoine biosynthesis operon. The recombinant strain pHM38 is a modified *H. marinus* strain that expresses GFP. The recombinant strain pHM42 is a modified *H. marinus* strain that carries an empty plasmid. The recombinant strains pHM38 and pHM42 serve as controls, as they contain only native ectoine biosynthesis genes. Error bars show standard deviation of n=3 technical replicates.

### EXAMPLES

The present invention is further described in more detail by the following examples which are only provided for illustrating the invention and which are not to be construed as limiting the scope of the invention. The following material and methods were used in the Examples.

### Example 1: Production of ectoine in the wild-type H. marinus MH-110

*H. marinus* strain MH-110 is obtainable from the Japanese Cell Collection (JCM) under deposition number JCM 7688 or from the Leibniz Institute DSMZ-German Collection of Microorganisms and Cell Cultures GmbH (DSMZ) under deposition number DSM 11271. The strain was cultured in a Basal Medium that was modified from Nishihara et al., 1991b with a final composition of 2 g/L K₂HPO₄, 1 g/L KH₂PO₄, 5 g/L (NH₄)₂SO₄, 60 g/L NaCl, 0.2 g/L MgSO₄ ▪ 7H₂O, 10 mg/L CaCl₂, 10 mg/L FeSO₄ ▪ 7H₂O, 0.6 mg/L NiSO₄ ▪ 7H₂O and 2 mL/L trace metal solution containing 1.68 mg/L Na₂MoO₄ ▪ 2H₂O, 7 mg/L ZnSO₄ ▪ 7H₂O, 0.5 mg/L CuSO₄ ▪ 5H₂O, 1 mg/mL H₃BO₃, 0.66 mg/L MnSO₄ ▪ 5H₂O, 1 mg/L CoCl₂ ▪ 6H₂O. *H. marinus* strain MH-110 was inoculated to an OD₅₄₀ = 0.05 and cultivated as 50 mL culture in a 300 mL Erlenmeyer baffled flask for 48 hrs. The Erlenmeyer flask was placed into a 2L anaerobic jar (anaerobic jar "small", Cat.-No. 3.380.202, Schuett-Biotec GmbH, Germany) filled with a gaseous atmosphere of H₂, CO₂ and O₂ in the ratio H₂:CO₂:O₂ = 87: 10:3. The jar was placed in an incubator set at 37°C and 150 rpm. The gas atmosphere was renewed after 24 and 42 hrs. The optical density of the cultures was measured using a photometer set to a wavelength of 540 nm (OD₅₄₀). The culture reached an OD₅₄₀ = 1.636. The culture was centrifuged at 4000xg for 10 min and concentrated to an OD₅₄₀=5 by partially removing the supernatant. The supernatant was stored at -20°C. Cell pellets according to OD₅₄₀=5 were collected by centrifugation of 1 mL of the concentrated culture at 5000xg for 3 min and cell pellets were stored at -20 °C until further analysis.

Approx. 200 µL silica beads (size 0.1-0.2 mm) and 500 µl ddH₂O were added to the cell pellets and cells were disrupted via bead beating with a FastPrep device (MP Biomedicals Germany GmbH) using three cycles at 7.5 m/s for 45 sec with 5 min breaks on ice. The lysates were collected. 150 µl of the cell lysate or culture supernatant were mixed with 600 µl EtOH and incubated at -80°C for >2 h. The samples were then centrifuged at 21.000xg and 4°C for 20 min. The supernatants were collected. Samples were filtered through 4 mm CA syringe filters with a pore size of 0.22 µm (Chromatographie Handel Müller GmbH).

Ectoine was quantified via HPLC using an Agilent 1260 Infinity system. 5 µl of each sample were loaded on a ReproSil-Pur column C18-AQ, 5 µm, 250x4.6 mm, (Dr. Maisch GmbH Ammerbuch-Entringen) kept at 40°C. The mobile phase was an isocratic mixture of 80% acetonitrile and 20% H₂O and a flow rate of 0.8 mL/min. Ectoine was detected at a retention time of 7.5 - 8 min. For quantification, the area under the curve at this retention time was referenced to ectoine standards prepared and analyzed under the same conditions. The standard curve is linear in the range of 0.001-1 mg/mL ectoine.

Results: The results are shown in Figure 1. *H. marinus* produces ectoine in the range of 30-35 µg/(mL^{∗}OD₅₄₀), i.e. one milliliter of a culture at OD₅₄₀ = 1 contains 30-35 µg ectoine. At a conversion factor of 0.55 g/L dry biomass per unit OD₅₄₀ this refers to a production range of ectoine of 55-64 mg ectoine per g biomass, which is 5.5-6.4% ectoine in dry weight. About 95% of the total ectoine is found in the cell lysate. As can be seen in Figure 1, only a small fraction of about 5% of ectoine is released into the media during the cultivation period.

### Example 2: NaCl-regulated production of ectoine in the wild-type H. marinus MH-110

The *H. marinus* wild-type strain MH-110 was cultivated as described in Example 1 with the following modifications: The NaCl concentration of the Basal Medium was varied in the range of 12.5 g/L - 60 g/L. Additionally, one culture containing 12.5 g/L NaCl was supplemented with 20 g/L Na₂SO₄. *H. marinus* was cultivated in small culture volume (1 mL) in 24-well cell culture plates. To prevent cells from forming aggregates, a glass bead of ca. 3 mm diameter was added to each 1 mL culture. The cultures were inoculated to an OD₅₄₀=0.024 and cultivated for 70 hours as described in Example 1. The gas atmosphere was renewed every 24 hrs. The cultures were centrifuged at 16.000xg for 1 min and cell pellet and culture supernatant were collected. Sample processing and HPLC quantification were performed as described in Example 1.

Results: The results are shown in Figure 2. It was found that *H. marinus* strain MH-110 produces ectoine if the NaCl concentration in the medium is 20 g/L or higher. Ectoine concentration increases with increasing NaCl concentration. When NaCl is partially substituted with Na₂SO₄, no ectoine is produced, indicating that chloride seems to be required for ectoine production.

### Example 3: Ectoine production in wild-type H. marinus MH-110 in modified DSMZ81

*H. marinus* wild-type strain MH-110 was cultured in a modified version of the DMSZ81 medium (Leibniz Institute DSMZ-German Collection of Microorganisms and Cell Cultures GmbH). The medium contains 2.9 g/L Na₂HPO₄ ▪ 2H₂O, 2.3 g/L KH₂PO₄, 2 g/L NH₄Cl, 0.5 g/L MgSO₄ ▪ 7H₂O, 10 mg/L CaCl₂ ▪ 2H₂O, 0.05 g/L ferric ammonium citrate, 0.5 mg/L ZnSO₄ ▪ 7H₂O; 0.15 mg/L MnCl₂ ▪ 4H₂O, 1.5 mg/L H₃BO₃, 1 mg/L CoCl₂ ▪ H₂O, 0.05 mg/L CuCl₂ ▪ 2H₂O, 0.1 mg/L NiCl₂ ▪ 6H₂O and 0.15 mg/L Na₂MoO₄ ▪ 2H₂O. NaCl was added in a concentration ranging from 15 - 60 g/L, or 15 g/L NaCl and 15 g/L Na₂SO₄ were added.

Results: The results are shown in Figure 3. It was found that *H. marinus* strain MH-110 produces ectoine if cultured in modified DSMZ81 medium, provided that the NaCl concentration in the medium is 20 g/L or higher. Production increases with increasing NaCl concentration. When NaCl is partially substituted by Na₂SO₄, no ectoine is produced. Therefore, similar results were observed in modified DSMZ81 medium and modified Basal Medium (see Example 1).

### Example 4: Simultaneous production of ectoine and single cell protein (SCP) in wild-type H. marinus MH-110 strains

*H. marinus* wild-type strain MH-110 was cultivated in Basal Medium (see Example 1) with a final composition of 2 g/L K₂HPO₄, 1 g/L KH₂PO₄, 5 g/L (NH₄)₂SO₄, 60 g/L NaCl, 0.2 g/L MgSO₄ ▪ 7H₂O, 10 mg/L CaCl₂, 10 mg/L FeSO₄ ▪ 7H₂O, 0.6 mg/L NiSO₄ ▪ 7H₂O and 2 mL/L trace metal solution containing 1.68 mg/L Na₂MoO₄ ▪ 2H₂O, 7 mg/L ZnSO₄ ▪ 7H₂O, 0.5 mg/L CuSO₄ ▪ 5H₂O, 1 mg/mL H₃BO₃, 0.66 mg/L MnSO₄ ▪ 5H₂O, 1 mg/L CoCl₂ ▪ 6H₂O. *H. marinus* strain MH-110 was inoculated to an OD₅₄₀ = 0.05 and cultivated as 50 mL culture in 300 mL Erlenmeyer baffled flask for 48 hrs. The Erlenmeyer flask was placed into a 2L anaerobic jar (anaerobic jar "small", Cat.-No. 3.380.202, Schuett-Biotec GmbH, Germany) which was filled with a gaseous atmosphere of H₂, CO₂ and O₂ at the ratio H₂:CO₂:O₂ = 87: 10:3. The jar was placed in an incubator set at 37°C, 150 rpm. The gas atmosphere was renewed after 24 and 42 hrs. The optical density of the cultures was measured using a photometer set to a wavelength of 540 nm (OD₅₄₀). The culture reached an OD₅₄₀ = 1.64. The culture was centrifuged at 4000xg for 10 min and concentrated to an OD₅₄₀=5 by removing supernatant. 1 mL samples of the culture were centrifuged at 5000xg for 3 min. The supernatant was collected and the cell pellet was resuspended in 1 mL of fresh medium containing 15 g/L NaCl instead of 60 g/L, to "flush out" the ectoine. The resuspended cell pellets were transferred into a 24-well plate containing a glass bead per well and cultivated under a gas atmosphere as described in Example 2. Cultures were centrifuged at >16.000xg for 1 min and cell pellet and culture supernatant were collected. Samples were processed and ectoine was quantified as described in Example 1. Protein content was quantified with Protein Assay Dye Reagent Concentrate (BioRad) according to the manufacturer's instructions.

Results: The results are shown in Figure 4 and 5. Before the flush step, almost all ectoine (95%) is found in the cell pellet. By transferring the pellet from a high-salt medium (60 g/L NaCl) to low-salt medium (15 g/L NaCl) and cultivation under autotrophic conditions as described above, 94% of the ectoine is flushed out of the cells, i.e. almost all ectoine (see Figure 4). Before the osmotic down-shock, 98% of the total protein is found in the cell lysate. After flush, a small portion of protein is found in the culture medium. However, the main body (89%) of protein remains in the cell (see Figure 5). This experiment indicates that the cells are not extensively lysed by the osmotic down-shock. The flush-step therefore allows separating ectoine and protein-rich biomass in one step. The proteins mostly remain in the cell pellet, which can be separated from the supernatant by centrifugation. Ectoine can be purified from the supernatant.

### Example 5: Simultaneous production of ectoine and single cell protein (SCP) in genetically modified H. marinus MH-110 strains

Recombinant *H. marinus* strains were prepared as described below. PCR-amplified gene fragments spanning different regions of the ectoine biosynthesis operon were cloned into the pHM38-, pHM39- or pHM40-based expression vectors containing different RBS and synthetic promoters. Additionally, plasmids were created expressing the ectoine biosynthesis genes under the control of the endogenous *ectA* promoter region from *H. marinus, Pect,* spanning the region from nucleotide 727 to nucleotide 1 upstream of the start codon of *ectA.* The following primers were used:
DNA_287 (SEQ ID NO: 12)
   5'-TGTCTTACTAGTATGGAGAGTCATACCGAAACC-3'
DNA_288 (SEQ ID NO: 13)
   5'-TGTCTTGGATCCTTATTCCTTCACCAAAGTATAAGAACC-3'
DNA_289 (SEQ ID NO: 14)
   5'-TGTCTTGGATCCTTCTTTAGTGTTCCCATATCGC-3'
DNA_290 (SEQ ID NO: 15)
   5'-TGTCTTGGATCCCTAGTTACTAGCTTGCTTTTGAAG-3'
DNA_291 (SEQ ID NO: 16)
   5'-TGTCTT GAATTCGATGAAAAATTCGTTGCTTCGG-3'
DNA_330 (SEQ ID NO: 17)
   5'-TGTCTTACTAGTATGAAAACTTTTGAACTGAATGAATCC-3'
DNA_331 (SEQ ID NO: 18)
   5'-TACCTTGGATCCTCAGGAAGCTTGGTTCTCG-3'

Plasmid pHM187 was constructed to include the endogenous ectoine biosynthesis genes *ectA, ectB* and *ectC* under the control of the synthetic constitutive promoter J23111 and the synthetic RBS BBa_B0064. The biosynthesis genes *ectA, ectB, ectC* were amplified from the *H. marinus* genome using primers DNA_287 and DNA_288. The fragment was digested with *Bcu*I/*Bam*HI and cloned into the backbone pHM39 digested with *Bcu*I/*Bam*HI*.*

Plasmid pHM188 was constructed to include the endogenous ectoine biosynthesis genes *ectA, ectB, ectC* and *ask* under the control of the synthetic constitutive promoter J23111 and the synthetic RBS BBa_B0064. The biosynthesis genes *ectA, ectB, ectC* and *ask* were amplified from the *H. marinus* genome using primers DNA_287 and DNA_289. The fragment was digested with *Bcu*I/*Bam*HI and cloned into the backbone pHM39 digested with *Bcu*I/*Bam*HI.

Plasmid pHM189 was constructed to include the endogenous ectoine biosynthesis genes *ectA, ectB, and ectC* under the control of the native promoter *Pect* and the native RBS. The biosynthesis genes *ectA, ectB, ectC* and the native promoter and RBS were amplified from the *H. marinus* genome using primers DNA_291 and DNA_288. The fragment was digested with *Eco*RI/*Bam*HI and cloned into the backbone pHM38 digested with *Eco*RI/*Bam*HI*.*

Plasmid pHM190 was constructed to include the endogenous ectoine biosynthesis genes *ectA, ectB, ectC* and *ask* under the control of the native promoter *Pect* and the native RBS. The biosynthesis genes *ectA, ectB, ectC* and *ask* as well as the native promoter and RBS were amplified from the *H. marinus* genome using primers DNA_291 and DNA_289. The fragment was digested with *Eco*RI/*Bam*HI and cloned into the backbone pHM38 digested with *Eco*RI/*Bam*HI*.*

Plasmid pHM191 was constructed to include the endogenous ectoine biosynthesis genes *ectA, ectB* and *ectC* under the control of the synthetic constitutive promoter J23114 and the synthetic RBS BBa_B0031. The biosynthesis genes *ectA, ectB, ectC* were amplified from the *H. marinus* genome using primers DNA_287 and DNA_288. The fragment was digested with *Bcu*I/*Bam*HI and cloned into the backbone pHM40 digested with *Bcu*I/*Bam*HI*.*

Plasmid pHM194 was constructed to include the endogenous ectoine biosynthesis genes *ectA, ectB, ectC, ask, HVMH_0179* and *HVMH_0180* under the control of the native promoter *Pect* and the native RBS. The biosynthesis genes and the native promoter and RBS were amplified from the *H. marinus* genome using primers DNA_291 and DNA_290. The fragment was digested with *Eco*RI/*Bam*HI and cloned into the backbone pHM38 digested with *Eco*RI/*Bam*HI*.*

Plasmid pHM204 was constructed to include the *ectB* gene PSTAA_0243 of *Pseudomonas stutzeri* DSM4166 under the control of the synthetic constitutive promoter J23111 and the synthetic RBS BBa_B0064. The *ectB* gene PSTAA_0243 of *P. stutzeri* DSM4166 was amplified from *P. stutzeri* DSM4166 genomic DNA using primers DNA_330 and DNA_331. The fragment was digested with *BcuI*/*BamHI* and cloned into the plasmid pHM39 digested with *Bcu*I/*Bam*HI*.*

Plasmid pHM206 was constructed to include the *ectB* gene PSTAA_0243 of *P. stutzeri* DSM4166 under the control of the synthetic constitutive promoter J23114 and the synthetic RBS BBa_B0031. The *ectB* gene PSTAA_0243 of *P. stutzeri* DSM4166 was amplified from *P. stutzeri* DSM4166 genomic DNA using primers DNA_330 and DNA_331. The fragment was digested with *Bcu*I/*Bam*HI and cloned into the plasmid pHM40 digested with *Bcu*I/*Bam*HI*.*

**Table 1: Overview on the genetically modified strains H. marinus used in the Example. HM: genes from H. marinus. PS: genes from P. stutzeri.**

| **Name** | **Reference** | **Description** |
|---|---|---|
| pHM38 | SEQ ID NO:2 | GFP with strong constitutive promoter (J23119), control |
| pHM42 | SEQ ID NO:5 | Empty plasmid, control |
| pHM187 | SEQ ID NO:19 | *ectABC(HM)* with moderate constitutive promoter (J23111) |
| pHM188 | SEQ ID NO:20 | *ectABC_ask(HM)* with moderate constitutive promoter (J23111) |
| pHM189 | SEQ ID NO:21 | *ectABC(HM)* with native promoter (P*_{ect}*) |
| pHM190 | SEQ ID NO:22 | *ectABC_ask(HM)* with native promoter (P*_{ect}*) |
| pHM191 | SEQ ID NO:23 | *ectABC(HM)* with weak constitutive promoter (J23114) |
| pHM194 | SEQ ID NO:24 | *ect_operon(HM)* with native promoter (P*_{ect}*) |
| pHM204 | SEQ ID NO:25 | *ectB(PS)* with moderate constitutive promoter (J23111) |
| pHM206 | SEQ ID NO:26 | *ectB(PS)* with weak constitutive promoter (J23114) |

The recombinant *H. marinus* strains were grown in modified basal medium as described in Example 1 with NaCl concentrations of 15, 30 and 60 g/L and with 15 µg/ml kanamycin. Strains were inoculated to an OD₅₄₀ = 0.05 and cultivated in 24-well plates as described in Example 2 for 3 days. Cells and supernatants were harvested, processed and ectoine was quantified both in the supernatant and in the cells as described in Example 1. Protein levels in the cells were determined by use of the Protein Assay Dye Reagent Concentrate (BioRad) according to the manufacturer's instructions. The recombinant strains harboring pHM38 and pHM42 were used as controls, since their ectoine biosynthesis is not modified. They can be cultivated in the same kanamycin-containing media as the recombinant strains.

Results: The results are shown in Figures 6-8. The production of ectoine of all recombinant strains was higher compared to the controls at 30 g/L and 60 g/L NaCl, as can be seen in Figure 6. For example, strain pHM187 produces 2.6-fold more ectoine at 60 g/L NaCl than the controls pHM38 and pHM42 under the same conditions. Strain pHM206, which expresses *P*. *stutzeri ectB* with the constitutive promoter J23114, shows the highest total ectoine production at 60 g/L NaCl which is 3.4-fold higher compared to the control strains under the same conditions. Thus, the recombinant strains produce 10-20% ectoine in dry weight at 60 g/L NaCl, which is a significant improvement to the 6.5% ectoine that is produced by the control strains carrying pHM38/pHM42 or the wild-type strain (Example 2). In addition, Figure 7 shows that all recombinant strains overexpressing ectoine biosynthesis genes show increased ectoine levels in the culture supernatant compared to the controls. In the recombinant cell cultures, most of the ectoine is secreted directly into the supernatant. Therefore, these strains secrete ectoine into the culture medium without the need of an additional osmotic shock step. None of the recombinant strains was associated with increased protein levels in the culture supernatant, indicating that these strains do not have an increased tendency to undergo cell lysis compared to the control strains (see Figure 8). This indicates that overexpression of the tested ectoine biosynthesis genes does not harm the respective host organism.

### Example 6: Production of bacterial biomass in wild-type H. marinus MH-110

*H. marinus* wild-type strain MH-110 was cultivated in modified DSMZ81 medium (described in Example 3) containing 15 g/L NaCl, in a continuous fermentation in a 15 Liter bioreactor under autotrophic conditions using H₂, CO₂ and ambient air. The cells were continuously harvested, collected and subsequently freeze-dried. The freeze-dried powder was used as a feed additive. Specifically, the powder was used to replace 30% (w/w) of a standard shrimp feed formulation, and 30% (w/w) or 60% (w/w) of a standard salmon feed formulation, respectively. The modified feed was fed to Atlantic Salmon (*Salmo salar*) and Whiteleg Shrimp (*Lito-penaeus vannamei*), respectively.

Results: It was found that the feed formulations containing up to 60% of the bacterial biomass were tolerated by the animals without any welfare concerns. This demonstrates that *Hydrogenovibrio* biomass can be used as feed additive for feeding shrimps and fish.

### LITERATURE

Nishihara, H., Igarashi, Y., and Kodama, T. (1989) Archives of Microbiology 152, 39-43
Arai, H., and Ishii, M. (2019) Microbiol Resour Announc 8
Jo, B. H., Hwang, B. H., and Cha, H. J. (2014) J Biotechnol 185, 37-38
Toyoda, K., Ishii, M., and Arai, H. (2018) J Biosci Bioeng 126, 730-735
Toyoda, K., Yoshizawa, Y., Arai, H., Ishii, M., and Igarashi, Y. (2005) Microbiology 151, 3615-3625
Cantera S., Tamarit, D., Strong, P. J., Sánchez-Andrea, I., Ettema T. J. G. & Sousa, D. Z. (2022), Reviews in Environmental Science and Bio/Technology, 21, 571-581
Hoff, B., Plassmeier, J., Blankschien, M., Letzel, A. C., Kourtz, L., Schroder, H., Koch, W., andZelder, O. (2021) Angew Chem Int Ed Engl 60, 2258-2278
Kachel, B., and Mack, M. (2020) Metab Eng
Nishihara, H., Igarashi, Y., and Kodama, T. (1991a) Journal of fermentation and bioengineering 72, 358-361
Nishihara, H., Igarashi, Y., and Kodama, T. (1991b) International Journal of Systematic Bacteriology, 41, 130-133
Nishihara, H., Miyashita, Y., Aoyama, K., Kodama, T., Igarashi, Y., and Takamura, Y. (1997) Biochem Biophys Res Commun 232, 766-770
Nishihara, H., Miyata, Y., Miyashita, Y., Bernhard, M., Pohlmann, A., Friedrich, B., and Takamura, Y. (2001) Biosci Biotechnol Biochem 65, 2780-2784
Nishihara, H., Yaguchi, T., Chung, S. Y., Suzuki, K., Yanagi, M., Yamasato, K., Kodama, T., and Igarashi, Y. (1998) Arch Microbiol 169, 364-368
Kim, J. Y., Jo, B. H., and Cha, H. J. (2011) J Biotechnol 155, 312-319
Bacher, A., Eberhardt, S., Eisenreich, W., Fischer, M., Herz, S., Illarionov, B., Kis, K., and Richter, G. (2001) Vitam Horm 61, 1-49
Fischer, M., and Bacher, A. (2005) Nat Prod Rep 22, 324-350
Joosten, V., and van Berkel, W. J. (2007) Curr Opin Chem Biol 11, 195-202
Stahmann, K. P., Revuelta, J. L., and Seulberger, H. (2000) Appl Microbiol Biotechnol 53, 509-516
Schwechheimer, S. K., Becker, J., Peyriga, L., Portais, J. C., Sauer, D., Muller, R., Hoff, B., Haefner, S., Schroder, H., Zelder, O., and Wittmann, C. (2018) Metab Eng 47, 357-373
Abbas, C. A., and Sibirny, A. A. (2011) Microbiol Mol Biol Rev 75, 321-360
Schwechheimer, S. K., Park, E. Y., Revuelta, J. L., Becker, J., and Wittmann, C. (2016) Appl Microbiol Biotechnol 100, 2107-2119
Averianova, L. A., Balabanova, L. A., Son, O. M., Podvolotskaya, A. B., and Tekutyeva, L. A. (2020) Front Bioeng Biotechnol 8, 570828
Perkins, J., and Pero, J. (2002) Biosynthesis of riboflavin, biotin, folic acid, and cobalamin. in Bacillus subtilis and Its Closest Relatives: from Genes to Cells (Sonenshein, A., Hoch, J., and Losick, R. eds.), ASM Press, Washington DC. pp 271-286
Acevedo-Rocha, C. G., Gronenberg, L. S., Mack, M., Commichau, F. M., and Genee, H. J. (2018) Curr Opin Biotechnol 56, 18-29
Mack, M., van Loon, A. P., and Hohmann, H. P. (1998) J Bacteriol 180, 950-955
Winkler, W. C., Cohen-Chalamish, S., and Breaker, R. R. (2002) Proc Natl Acad Sci USA 99, 15908-15913
Mironov, A. S., Gusarov, I., Rafikov, R., Lopez, L. E., Shatalin, K., Kreneva, R. A., Perumov, D. A., and Nudler, E. (2002) Cell 111, 747-756
Pedrolli, D. B., Kuhm, C., Sevin, D. C., Vockenhuber, M. P., Sauer, U., Suess, B., and Mack, M. (2015) Proc Natl Acad Sci USA 112, 14054-14059
Haase, I., Grawert, T., Illarionov, B., Bacher, A., and Fischer, M. (2014) Methods Mol Biol 1146, 15-40
Chen et al. 2017 World J Microbiol Biotechnol., 33(6): 116.
Hemberger, S., Pedrolli, D. B., Stolz, J., Vogl, C., Lehmann, M., and Mack, M. (2011) BMC Biotechnol 11, 119
Schwarz, J., Konjik, V., Jankowitsch, F., Sandhoff, R., and Mack, M. (2016) Angew Chem Int Ed Engl 55, 6103-6106
Pedrolli, D. B., Jankowitsch, F., Schwarz, J., Langer, S., Nakanishi, S., and Mack, M. (2014) Methods Mol Biol 1146, 41-63
Jankowitsch, F., Schwarz, J., Ruckert, C., Gust, B., Szczepanowski, R., Blom, J., Pelzer, S., Kalinowski, J., and Mack, M. (2012) J Bacteriol 194, 6818-6827
Haase, I., Sarge, S., Illarionov, B., Laudert, D., Hohmann, H. P., Bacher, A., and Fischer, M. (2013) Chembiochem 14, 2272-2275
Sarge, S., Haase, I., Illarionov, B., Laudert, D., Hohmann, H. P., Bacher, A., and Fischer, M. (2015) Chembiochem 16, 2466-2469
Olson, K., Fenno, J., Lin, N. et al. (1981) Nature 293, 408-411
Bishé, B., Arnaud, T., and Golden, J. W. (2019), Iscience 20, 216-228
Louis, P; Galinski, E. A. (1997), Microbiology. 143 (4): 1141-9
Case & Atsumi (2016), J Biotechnol, 231, 106-114
Daniel E., Onwukwe, G. U., Wierenga, R. K., Quaggin S. E., Vainio S. J. and Krause, M. (2015), BMC Bioinformatics, 16, Article number: 303

## Claims

1. Method for producing ectoine, comprising
(a) providing chemolithoautotrophic bacterium of the genus *Hydrogenovibrio;*
(b) culturing the bacterium in a culture medium that allows the propagation of the bacterium and contains at least 20 g/L NaCl;
(c) incubating the bacterium in a culture medium having a NaCl content of less than 20 g/L NaCl;
(d) obtaining the ectoine from the culture medium; and
(e) optionally, obtaining the cells from the culture medium.

2. Method of claim 1, wherein the chemolithoautotrophic bacterium is of the species *Hydrogenovibrio marinus.*

3. Method of claim 2, wherein the chemolithoautotrophic bacterium is derived from *Hydrogenovibrio* marinus MH-110, deposited at the Japanese Cell Collection (JCM) under deposition number JCM 7688, or at the the Leibniz Institute DSMZ-German Collection of Microorganisms and Cell Cultures GmbH (DSMZ) under deposition number DSM 11271.

4. Method of any of claims 1-3, wherein step (b) comprises culturing the bacterium in a gas atmosphere that comprises H₂, CO₂, O₂ and N₂.

5. Method of any of claims 1-4, wherein the medium used in step (b) contains at least 30 g/L NaCl, and preferably at least 40 g/L NaCl.

6. Method of any of claims 1-5, wherein the medium used in step (c) contains a NaCl content of less than 15 g/L NaCl, and preferably less than 10 g/L NaCl.

7. Method of any of claims 1-6, wherein step (e) further comprises obtaining the proteins from the cells.

8. Method of any of claims 1-7, wherein the bacterium is a naturally occurring bacterium which has not been genetically modified.

9. Method of any of claims 1-7, wherein the bacterium has been genetically modified to overexpress one or more endogenous or exogenous ectoine biosynthesis genes.

10. Method of claim 9, wherein the bacterium has been genetically modified to overexpress one or more of the endogenous or exogenous biosynthesis genes *ectA, ectB,* and *ectC.*

11. Method of claim 10, wherein the bacterium has been genetically modified to overexpress the endogenous or exogenous biosynthesis gene *ectB,* but has not been genetically modified to overexpress the endogenous or exogenous biosynthesis genes *ectA* and *ectC.*

12. Chemolithoautotrophic bacterium of the genus *Hydrogenovibrio* which has been genetically modified to overexpress one or more endogenous or exogenous ectoine biosynthesis genes.

13. Chemolithoautotrophic bacterium of claim 12, wherein the bacterium has been genetically modified to overexpress one or more of the endogenous or exogenous biosynthesis genes *ectA, ectB,* and *ectC.*

14. Chemolithoautotrophic bacterium of claim 13, wherein the bacterium has been genetically modified to overexpress the endogenous or exogenous biosynthesis gene *ectB,* but has not been genetically modified to overexpress the endogenous or exogenous biosynthesis genes *ectA* and *ectC.*

15. Use of the chemolithoautotrophic bacterium of any of claims 12-14 for the production of ectoine.
